# EUROPEAN PATENT SPECIFICATION

(11) **EP 2 432 496 B1**
(45) Date of publication and mention of the grant of the patent: **28.03.2018**
(21) Application number: 10722931.2
(22) Date of filing: 06.05.2010
(51) Int. Cl.: A61K 38/44, C12N 9/06, C12N 15/63, C12Q 1/68

(54) **CONTROL OF URIC ACID HOMEOSTASIS**
KONTROLLE VON HARNSÄURE-HOMÖOSTASE
RÉGULATION DE L'HOMÉOSTASIE DE L'ACIDE URIQUE

(30) Priority: 19.05.2009 EP 09006711
(43) Date of publication of application: 28.03.2012
(73) Proprietor: ETH Zurich, 8092 Zürich (CH)
(72) Inventor: KEMMER, Christian, CH-4125 Riehen (CH); WEBER, Wilfried, 79100 Freiburg (DE); FUSSENEGGER, Martin, CH-5506 Mägenwil / AG (CH)
(74) Representative: Latscha Schöllhorn Partner AG
(86) International application number: PCT/EP2010/002783
(87) International publication number: WO 2010/133298

(56) References cited:
- WO-A2-2008/002911
- US-A1- 2007 274 977
- WILKINSON STEVEN P ET AL: "HucR, a novel uric acid-responsive member of the MarR family of transcriptional regulators from Deinococcus radiodurans" JOURNAL OF BIOLOGICAL CHEMISTRY, AMERICAN SOCIETY FOR BIOCHEMISTRY AND MOLECULAR BIOLOGY, INC, US, vol. 279, no. 49, 3 December 2004 (2004-12-03), pages 51442-51450, XP009137045 ISSN: 0021-9258
- WILKINSON S P ET AL: "Negative Cooperativity of Uric Acid Binding to the Transcriptional Regulator HucR from Deinococcus radiodurans" JOURNAL OF MOLECULAR BIOLOGY, LONDON, GB LNKD- DOI:10.1016/J.JMB.2005.05.027, vol. 350, no. 4, 22 July 2005 (2005-07-22) , pages 617-630, XP004951603 ISSN: 0022-2836
- DEUSCHLE U ET AL: "TETRACYCLINE-REVERSIBLE SILENCING OF EUKARYOTIC PROMOTERS" MOLECULAR AND CELLULAR BIOLOGY, AMERICAN SOCIETY FOR MICROBIOLOGY, WASHINGTON, US, vol. 15, no. 4, 1 April 1995 (1995-04-01), pages 1907-1914, XP000564650 ISSN: 0270-7306
- HU M C-T ET AL: "THE INDUCIBLE LAC OPERATOR-REPRESSOR SYSTEM IS FUNCTIONAL IN MAMMALIAN CELLS" CELL, CELL PRESS, CAMBRIDGE, NA, US LNKD- DOI:10.1016/0092-8674(87)90234-0, vol. 48, no. 4, 27 February 1987 (1987-02-27), pages 555-566, XP001056394 ISSN: 0092-8674
- TERKELTAUB ROBERT ET AL: "Recent developments in our understanding of the renal basis of hyperuricemia and the development of novel antihyperuricemic therapeutics" ARTHRITIS RESEARCH THERAPY, BIOMED CENTRAL LTD, GB, vol. 8, no. Suppl.1, 1 January 2006 (2006-01-01), page S4, XP009137090 ISSN: 1478-6362
- KRAMER ET AL: "Semi-synthetic mammalian gene regulatory networks" METABOLIC ENGINEERING, ACADEMIC PRESS, US LNKD- DOI:10.1016/J.YMBEN.2005.02.005, vol. 7, no. 4, 1 July 2005 (2005-07-01), pages 241-250, XP005052635 ISSN: 1096-7176
- SEOANE A S ET AL: "CHARACTERIZATION OF MARR, THE REPRESSOR OF THE MULTIPLE ANTIBIOTIC RESISTANCE (MAR) OPERON IN ESCHERICHIA COLI" JOURNAL OF BACTERIOLOGY, AMERICAN SOCIETY FOR MICROBIOLOGY, WASHINGTON, DC; US, vol. 177, no. 12, 1 June 1995 (1995-06-01) , pages 3414-3419, XP000937842 ISSN: 0021-9193 & COHEN S P ET AL: "SALICYLATE INDUCTION OF ANTIBIOTIC RESISTANCE IN ESCHERICHIA COLI: ACTIVATION OF THE MAR OPERON AND A MAR-INDEPENDENT PATHWAY" JOURNAL OF BACTERIOLOGY, AMERICAN SOCIETY FOR MICROBIOLOGY, WASHINGTON, DC; US, vol. 175, no. 24, 1 December 1993 (1993-12-01), pages 7856-7862, XP008049292 ISSN: 0021-9193
- MEIMA ROB ET AL: "Promoter cloning in the radioresistant bacterium Deinococcus radiodurans" JOURNAL OF BACTERIOLOGY, AMERICAN SOCIETY FOR MICROBIOLOGY, WASHINGTON, DC; US LNKD- DOI:10.1128/JB.183.10.3169-3175.2001, vol. 183, no. 10, 1 May 2001 (2001-05-01), pages 3169-3175, XP002491110 ISSN: 0021-9193
- ENOMOTO A ET AL: "Molecular identification of a renal urate-anion exchanger that regulates blood urate levels" NATURE, NATURE PUBLISHING GROUP, LONDON, GB, vol. 417, no. 6887, 23 May 2002 (2002-05-23), pages 447-452, XP002314925 ISSN: 0028-0836
- DAVIS S ET AL: "HYPOURICAEMIC EFFECT OF POLYETHYLENEGLYCOL MODIFIED URATE OXIDASE" LANCET THE, LANCET LIMITED. LONDON, GB LNKD- DOI:10.1016/S0140-6736(81)90528-6, 8 August 1981 (1981-08-08), pages 281-283, XP000577404 ISSN: 0140-6736
- KEMMER CHRISTIAN ET AL: "Self-sufficient control of urate homeostasis in mice by a synthetic circuit" NATURE BIOTECHNOLOGY, NATURE PUBLISHING GROUP, NEW YORK, NY, US LNKD- DOI:10.1038/NBT.1617, vol. 28, no. 4, 1 April 2010 (2010-04-01), pages 355-360, XP009137044 ISSN: 1087-0156 [retrieved on 2010-03-28]
- COHEN S P ET AL: "GENETIC AND FUNCTIONAL ANALYSIS OF THE MULTIPLE ANTIBIOTIC RESISTANCE (MAR) LOCUS IN ESCHERICHIA COLI", JOURNAL OF BACTERIOLOGY, AMERICAN SOCIETY FOR MICROBIOLOGY, US, vol. 175, no. 5, 1 March 1993 (1993-03-01) , pages 1484-1492, XP002069168, ISSN: 0021-9193

### SEQUENCE LISTING

<110> ETH Zürich Kemmer, Christian weber, wilfried Fussenegger, Martin
<120> Control of uric acid homeostasis
<130> EM-T-09-026
<160> 6
<170> PatentIn version 3.4
<210> 1
   <211> 29
   <212> DNA
   <213> Artificial
<220>
   <223> Synthetic construct OCK20, probe for HucR
<400> 1
   ttggcgcgct cagcccgcat ggacaacga 29
<210> 2
   <211> 29
   <212> DNA
   <213> Artificial
<220>
   <223> Synthetic construct OCK21, probe for HucR
<400> 2
   gctctagatt ataccccctg ctccagccc 29
<210> 3
   <211> 31
   <212> DNA
   <213> Artificial
<220>
   <223> Synthetic construct OCK22, probe for KRAB
<400> 3
   ggaattccac catggatgct aagtcactaa c 31
<210> 4
   <211> 28
   <212> DNA
   <213> Artificial
<220>
   <223> Synthetic construct OCK23, probe for KRAB
<400> 4
   ttggcgcgcc cagagatcat tccttgcc 28
<210> 5
   <211> 38
   <212> DNA
   <213> Artificial
<220>
   <223> Synthetic construct OCK66, probe for muox
<400> 5
   ggaattccac catgtccgca gtaaaagcag cccgctac 38
<210> 6
   <211> 23
   <212> DNA
   <213> Artificial
<220>
   <223> Synthetic construct OCK67, probe for muox
<400> 6
   caagcttgca tgcaggcctc tgc 23

## Description

### Field of the invention

The invention relates to vectors and mammalian cells in a system useful for switching on or switching off gene expression in response to uric acid.

### Background of the invention

Uric acid homeostasis is essential for humans since this metabolite is an important radical scavenger and oxidative stress protectant reducing the development of cancer, Parkinson's disease and multiple sclerosis. However, due to the loss of urate oxidase during evolution, humans have a predisposition for hyperuricemia which favors the formation of urate crystal deposits associated with nephropathy, tumor lysis syndrome and gout.

Gout is the most common inflammatory arthritis affecting over 1% of the human population in industrialized countries and its prevalence is globally on the rise as the growing consumption of carbohydrates promotes obesity and hyperinsulinism, which reduces renal clearance of uric acid, the end product of purine metabolism. Because of the evolutionary loss of the urate oxidase, converting uric acid into the more soluble allantoin, blood uric acid levels are particularly high in humans, which improves oxidative stress resistance and might have been essential for hominid brain evolution by having prevented oxidative damage to the increasingly complex neuronal networks. High physiologic urate levels are typically well tolerated by humans since a urate transporter in the kidney regulates urate levels in the bloodstream. However, imbalances of urate homeostasis may lead to hyperuricemia and formation of pathologic monosodium urate crystal deposits in the joints, kidney and subcutaneous tissues which trigger urate-associated pathologies such as hypertension, cardiovascular disease, urate nephrolithiasis and gout.

*Deinococcus radiodurans* R1, which is among earth's most radiation-resistant organisms, has also evolved a remarkable ability to withstand other sources of DNA damage including ultraviolet radiation and oxidative stress (Daly, M.J., Nat. Rev. Microbiol. 7, 237-245 (2009)). Recently, a hypothetical uricase regulator (HucR) has been identified in *D. radiodurans* which was suggested to play a critical role in the cellular response to oxidative stress. Akin to mammals, *D. radiodurans* seems to take advantage of the radical scavenging activity of uric acid, yet needs to control its level to prevent crystallization. HucR was shown to bind to a dyad-symmetrical operator site (hucO) in the intergenic region of divergently transcribed *hucR* and a putative uricase suggesting that both genes are by default co-repressed by HucR, unless excessive uric acid levels trigger the release of HucR from hucO and induce uricase expression (Wilkinson, S.P. & Grove, A., J. Biol. Chem. 279, 51442-51450 (2004); J. Mol. Biol. 350, 617-630 (2005)).

### Summary of the invention

The invention relates to a mammalian cell useful in detecting and/or degrading a harmful excess of uric acid comprising
(a) a vector comprising a genetic code for a bacterial uric acid sensor-regulator fused to a a transrepressor domain, wherein the bacterial uric acid sensor-regulator is uricase sensor-regulator HucR from *Deinococcus radiodurans* R1, the MarR type transcriptional regulator Dgeo 2531 from *Deinococcus geothermalis* DSM 11300, the MarR-type transcriptional regulator from *Pseudomonas mendocina* ypm, the MarR-type transcriptional regulator Deide 3p00280 from *Deinococcus deserti* VCD115, or a sensor-regulator derived therefrom, wherein the sensor-regulator derived therefrom contains only conservative amino substitutions and remains at least 90% identical to the said bacterial uric acid sensor-regulator at the amino acid level and includes compounds at the polynucleotide level comprising triplet codons coding for the same amino acid but being especially adapted to mammalian cells; and
(b) a vector comprising an operator sequence specifically binding said bacterial uric acid sensor-regulator, a promoter and a polynucleotide coding for a protein, wherein the operator sequence is an operator sequence produced by *Deinococcaceae or Pseudomonadaceae* specifically binding to sensor-regulator HucR from *Deinococcus radiodurans* R1, Dgeo 2531 from *Deinococcus geothermalis* DSM 11300, the MarR-type transcriptional regulator from *Pseudomonas mendocina* ypm, or Deide 3p00280 from *Deinococcus deserti* VCD115, or an operator sequence derived therefrom, wherein the operator sequence derived therefrom is an operator sequence produced by *Deinococcaceae or Pseudomonadaceae* specifically binding to sensor-regulator HucR from *Deinococcus radiodurans* R1, Dgeo 2531 from *Deinococcus geothermalis* DSM 11300, the MarR-type transcriptional regulator from *Pseudomonas mendocina* ypm, or Deide 3p00280 from *Deinococcus deserti* VCD115, wherein one to twelve nucleotides of the operator
sequence are replaced by other nucleotides without diminishing the intended interaction with the sensor-regulator, wherein the protein is uricase or urate transporter protein.

In particular the invention relates to such a mammalian cell wherein the bacterial uric acid sensor-regulator is uricase sensor-regulator HucR from *Deinococcus radiodurans* R1, and the operator sequence is the corresponding operator sequence hucO from *Deinococcus radiodurans* R1.

Furthermore the invention relates to the mentioned mammalian cell in a nano- or microcontainer, a mammal excluding man comprising such a mammalian cell optionally in a nano- or microcontainer.

### Brief description of the Figures

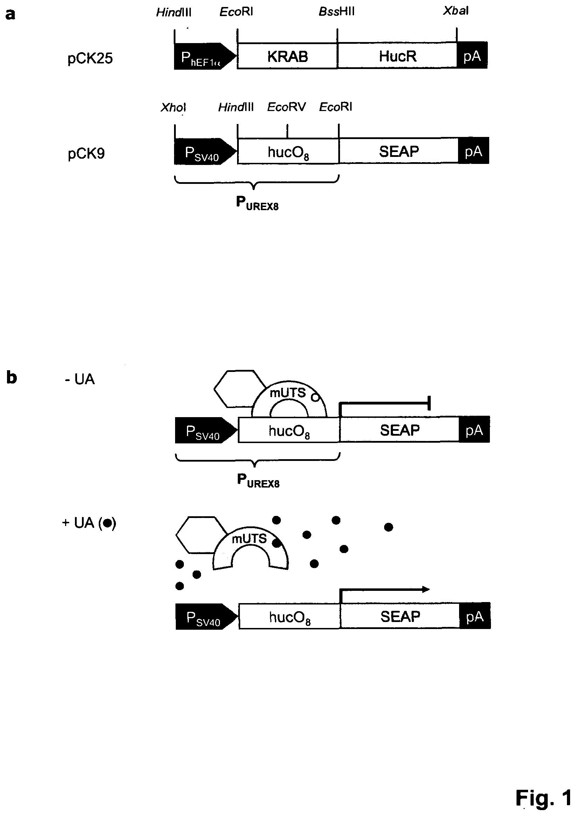

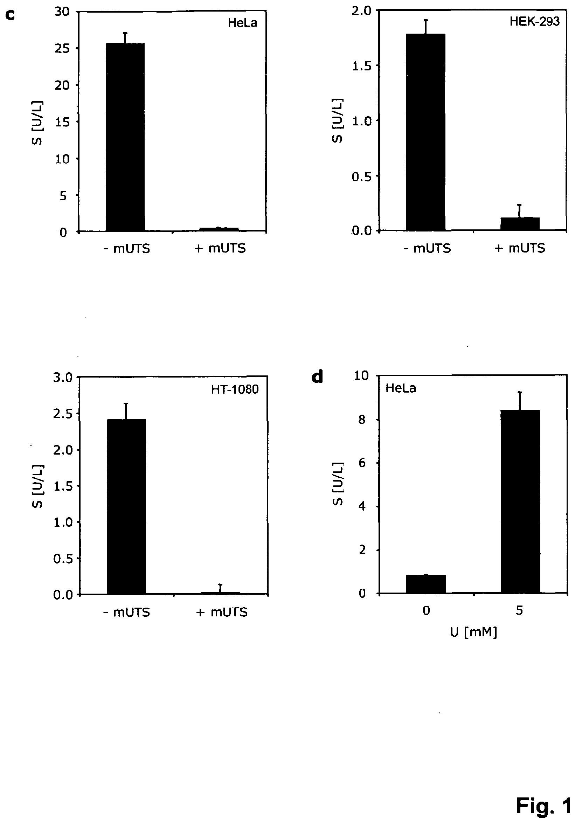

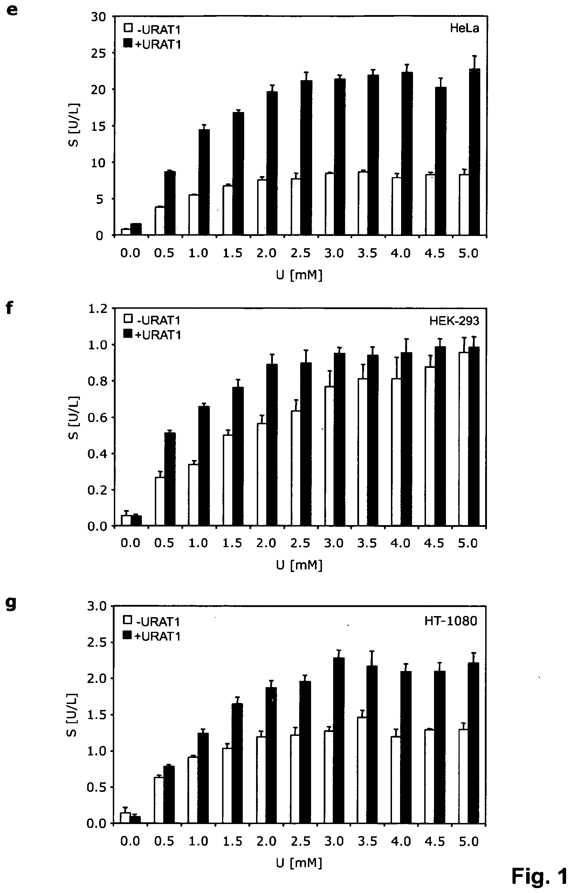

*Figure 1**.* Synthetic uric acid-responsive mammalian sensor circuit.
   (**a**) Expression vectors for uric acid-responsive transgene expression (UREX) (see Table 1 for abbreviations).
   (**b**) Diagram of UREX in action. In the absence of uric acid (-UA) mUTS (KRAB-HucR) binds to hucO₈ and represses SEAP production. In the presence of uric acid (+UA) mUTS is released from hucO₈ which derepresses P_{UREX8} and results in P_{SV40}-driven SEAP expression.
   (**c**) mUTS-mediated repression of P_{UREX8} in different human cell lines. HeLa, HEK-293 and HT-1080 were co-transfected with pCK9 (P_{UREX8}-SEAP-pA) and pCK25 (P_{hEF1α}-mUTS-pA) and cultivated for 48 h prior to quantification of SEAP expression.
   (**d**) Functional validation of the UREX sensor circuit. 4×10⁴ HeLa cells were co-transfected with the UREX expression vectors pCK9 and pCK25 (see Fig. 1a) and cultivated for 48 h in the presence or absence of 5 mM uric acid before SEAP was quantified in the cell culture supernatant.
   (**e-g**) Urate-dependent dose-response characteristics of UREX in the presence and absence of the human urate transporter URAT1. 3x10⁵ HeLa (**e**), HEK-293 (**f**) or HT-1080 cells (**g**) were co-transfected with the UREX sensor plasmids pCK9 and pCK25 (see Fig. 1a) and either pURAT1 (P_{hCMV}-URAT1-pA) or the isogenic control vector pcDNA3.1. The transfected populations were cultivated in medium supplemented with different uric acid concentrations and resulting SEAP levels were profiled after 48 h.
   (S) SEAP production; (U) uric acid concentration; (mUTS) mammalian urate transsilencer.

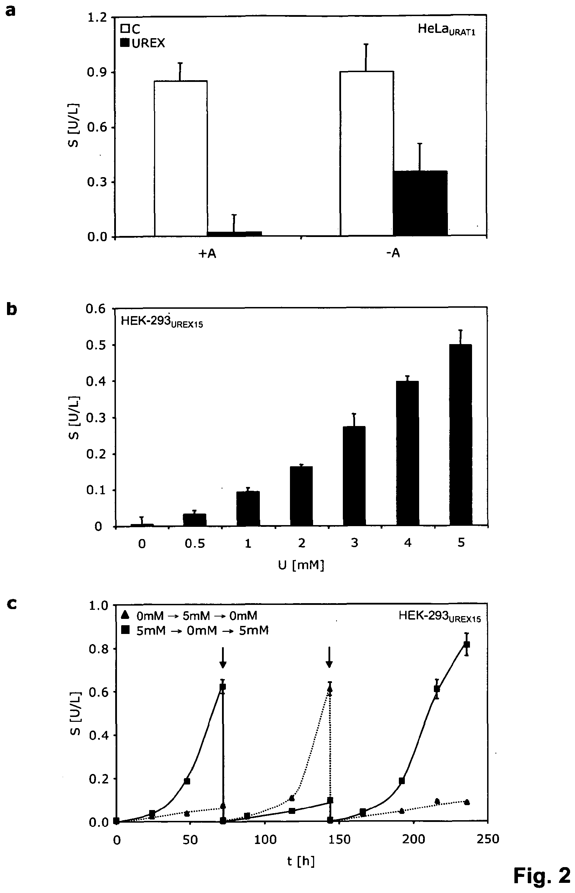

*Figure 2**.* Validation of UREX-controlled SEAP expression in urate oxidase-deficient mice and transgenic HEK-293.
   (**a**) HeLa_{URAT1} cells engineered for UREX-controlled SEAP expression were microencapsulated in coherent alginate-poly-L-lysine-alginate microcapsules and intraperitoneally injected (2×10⁶ cells per mouse, 200 cells/capsule) into (i) untreated *uox*^{*-*/*-*} mice exhibiting pathologic urate levels or (ii) into *uox*^{*-*/*-*} mice which had received 150 µg/mL (w/v) of the hyperuricemia therapeutic allopurinol in their drinking water to reduce urate levels (UREX). Control implants contained cells transgenic for constitutive SEAP expression (Control). SEAP levels were profiled in the serum of the animals 72 h after cell implantation.
   (**b**) Urate-based dose-response profile of the triple-transgenic HEK-293_{UREX15} cell line stably engineered for UREX-controlled SEAP and constitutive URAT1 expression. 5×10⁴ HEK-293_{UREX15} were cultivated in the presence of different urate concentrations and SEAP levels were quantified in the culture supernatant after 72 h.
   (**c**) Reversibility of the UREX-based uric acid sensor circuit. 2×10⁵ HEK-293_{UREX15} cells were cultivated for 10 days while alternating uric acid concentrations from 0 to 5 mM every 72 h (arrows).
   (C) control; (S) SEAP production; (U) uric acid concentration; (+A) +Allopurinol (low urate); (-A) -Allopurinol (high urate); (t) time.

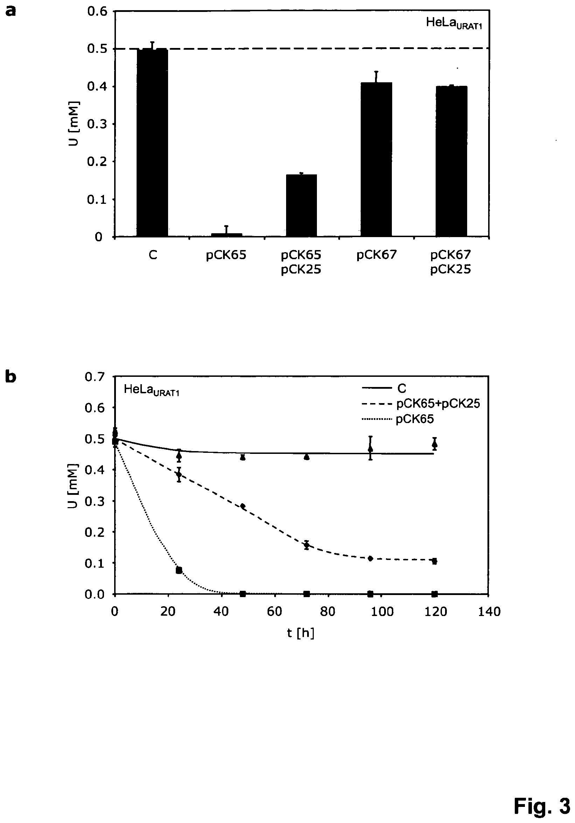

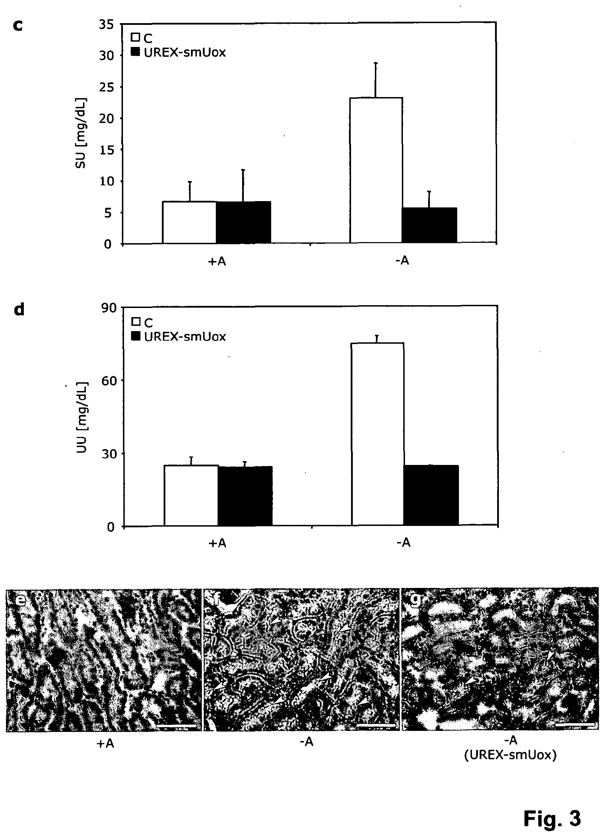

*Figure 3**.* Functional characterization of an engineered mammalian *Aspergillus flavus-*derived urate oxidase
   (**a**) Profiling of urate reduction mediated by constitutive or UREX-controlled expression of an intracellular (mUox) or secretion-engineered (smUox) urate oxidase variant. 2×10⁵ HeLa_{URAT1} cells were co-transfected with either (i) pCK65 (P_{UREX8}-smUox-pA) and isogenic control vector pcDNA3.1, (ii) pCK65 (P_{UREX8}-smUox-pA) and pCK25 (P_{hEF1α}-mUTS-pA), (iii) pCK67 (P_{UREX8}-mUox-pA) and pcDNA3.1 or (iv) pCK67 (P_{UREX8}-mUox-pA) and pCK25 (P_{hEF1α}-mUTS-pA). The cells were cultivated for 72 h with 0.5 mM uric acid before urate levels were determined in the culture supernatant.
   (**b**) Urate degradation profiles UREX-controlled smUox expression. 2×10⁵ HeLa_{URAT1} cells (solid line) engineered for constitutive (dotted line) or UREX-controlled smUox (dashed line) expression were cultivated in medium containing a starting uric acid concentration of 0.5 mM and uric acid reduction kinetics were profiled for 120 h.
   (**c,d**) UREX-controlled smUox-mediated reduction of pathologic uric acid levels in mice. Microencapsulated HeLa_{URAT1} cells engineered for UREX-controlled smUox expression were intraperitoneally implanted (2×10⁶ cells per mouse) into untreated *uox*^{*-*/*-*} mice exhibiting pathologic urate levels or into *uox*^{*-*/*-*} mice having received 150 µg/mL (w/v) of the hyperuricemia therapeutic allopurinol in their drinking water (UREX-smUox). Control implants contained parental HeLa_{URAT1} (Control). Uric acid levels were profiled in serum (**c**) and urine (collected for 24 h) (**d**) of the animals 72 h after cell implantation.
   (**e-g**) Tissue sections showing anisotropic monosodium urate crystal deposits (arrows) in the kidneys of (**e**) *uox*^{*-*/*-*} mice receiving 150µg/mL (w/v) allopurinol (positive control), (f) *uox*^{*-*/*-*} mice (negative control) and (**g**) *uox*^{*-*/*-*} mice implanted with HeLa_{URAT1} engineered for UREX-controlled smUox expression.
   (C) control; (U) uric acid concentration; (SU) serum uric acid; (UU) urinary uric acid; (+A) +Allopurinol (low urate); (-A) -Allopurinol (high urate); (t) time. Scale bars 100 µM.

### Detailed description of the invention

The herein described "UREX" system is a biosensor for self-sufficient auto-control of uric acid homeostasis. The system can be applied *in vitro* and *in vivo* as a general biosensor for the detection and response to uric acid. The UREX system can be used to either (i) switch on or (ii) switch off gene expression in response to uric acid in mammalian cells.

Uric acid is 7,9-dihydro-3H-purine-2,6,8-trione of the formula 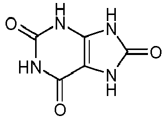 and exists in several tautomeric forms. Uric acid as used herein includes all tautomeric forms of uric acid and salts thereof, called "urates". Salts are, for example, monosodium urate or mono- or dipotassium urate. The invention relates to a mammalian cell useful in detecting and/or degrading a harmful excess of uric acid comprising
(a) a vector comprising a genetic code for a bacterial uric acid sensor-regulator fused to a a transrepressor domain, wherein the bacterial uric acid sensor-regulator is uricase sensor-regulator HucR from *Deinococcus radiodurans* R1, the MarR type transcriptional regulator Dgeo 2531 from *Deinococcus geothermalis* DSM 11300, the MarR-type transcriptional regulator from *Pseudomonas mendocina* ypm, the MarR-type transcriptional regulator Deide 3p00280 from *Deinococcus desert* VCD115, or a sensor-regulator derived therefrom, wherein the sensor-regulator derived therefrom contains only conservative amino substitutions and remains at least 90% identical to the said bacterial uric acid sensor-regulator at the amino acid level and includes compounds at the polynucleotide level comprising triplet codons coding for the same amino acid but being especially adapted to mammalian cells; and
(b) a vector comprising an operator sequence specifically binding said bacterial uric acid sensor-regulator, a promoter and a polynucleotide coding for a protein, wherein the operator sequence is an operator sequence produced by *Deinococcaceae or Pseudomonadaceae* specifically binding to sensor-regulator HucR from *Deinococcus radiodurans* R1, Dgeo 2531 from *Deinococcus geothermalis* DSM 11300, the MarR-type transcriptional regulator from *Pseudomonas mendocina* ypm, or Deide 3p00280 from *Deinococcus deserti* VCD115, or an operator sequence derived therefrom, wherein the operator sequence derived therefrom is an operator sequence produced by *Deinococcaceae or Pseudomonadaceae* specifically binding to sensor-regulator HucR from *Deinococcus radiodurans* R1, Dgeo 2531 from *Deinococcus geothermalis* DSM 11300, the MarR-type transcriptional regulator from *Pseudomonas mendocina* ypm, or Deide 3p00280 from *Deinococcus deserti* VCD115, wherein one to twelve nucleotides of the operator sequence are replaced by other nucleotides without diminishing the intended interaction with the sensor-regulator, wherein the protein is uricase or urate transporter protein.

A bacterial uric acid sensor-regulator is, for the purposes of the invention, derived from or related to natural uric acid sensor-regulators such as HucR proteins produced by *Deinococcaceae or Pseudomonadaceae.* A bacterial uric acid sensor-regulator is, for example, the transcriptional regulator HucR (DR_1159) from *Deinococcus radiodurans* R1, the MarR type transcriptional regulator Dgeo_2531 from *Deinococcus geothermalis* DSM 11300, the MarR-type transcriptional regulator from *Pseudomonas mendocina* ypm, the MarR-type transcriptional regulator Deide_3p00280 from *Deinococcus deserti* VCD115, and sensor-regulators derived from these mentioned naturally occurring sensor-regulators. By "derived from" a natural uric acid sensor-regulator such as HucR protein produced by *Deinococcaceae or Pseudomonadaceae,* is meant, in this context, that the amino acid sequence is identical to a naturally occurring sensor-regulator, or contains only conservative amino substitutions and remains at least 70%, preferably 80%, and more preferably 90% identical at the amino acid level. By "related to" a natural uric acid sensor-regulator such as HucR proteins produced by *Deinococcaceae or Pseudomonadaceae* is meant, for purposes of the invention, that the polynucleotide sequence that encodes the amino acid sequence hybridizes to a naturally occurring uric acid sensor-regulator under at least low stringency conditions, more preferably moderate stringency conditions, and most preferably high stringency conditions, and binds to a natural uric acid sensor-regulator recognition sequence. Conservative substitution is known in the art and described by Dayhof, M.D., 1978, Nat. Biomed. Res. Found., Washington, D.C., Vol. 5, Sup. 3, among others. Genetically encoded amino acids are generally divided into four groups: (1) acidic = aspartate, glutamate; (2) basic = lysine, arginine, histidine; (3) nonpolar = alanine, valine, leucine, isoleucine, proline, phenylalanine, methionine, tryptophan; and (4) uncharged polar = glycine, asparagine, glutamine, cysteine, serine, threonine, tyrosine. Phenylalanine, tryptophan and tyrosine are also jointly classified as aromatic amino acids. A substitution of one amino acid in a particular group with another amino acid in the same group is generally regarded as a conservative substitution. It is understood, for the purpose of this invention, that "derived from" and "related to" also includes compounds at the polynucleotide level comprising triplet codons coding for the same amino acid but being especially adapted to the intended host cell, e.g. mammalian cell. Such polynucleotides especially adapted to mammalian cells are particularly preferred.

A corresponding operator sequence specifically binding said bacterial uric acid sensor-regulator is hucO, the tandem repeat-containing operator site recognized by HucR. Other operator sequences considered are those operator sequences specifically binding the mentioned sensor-regulators produced by *Deinococcaceae or Pseudomonadaceae,* and operator sequences "derived from" and "related to" the mentioned operator sequences. Under "derived from" is understood, for the purpose of this invention, that one or more nucleotides, in particular one to twelve, such as one, two, three, four or five nucleotides, may be replaced by other nucleotides without diminishing the intended interaction with the sensor-regulator. An operator sequence "related to" according to the invention hybridizes to a naturally occurring operator-sequence under at least low stringency conditions, more preferably moderate stringency conditions, and most preferably high stringency conditions, and binds to a natural uric acid sensor-regulator. Particular operator sequences considered are tandem repeats of naturally occurring operator sequences or sequences derived therefrom comprising, for example, between 2 and 100, in particular between 5 and 20, repeats.

A transactivation domain is, for example, the *vp16* transactivation domain of *Herpes simplex* virus, the *p65* transactivation domain, the human e2f4 transactivation domain, and transactivation domains derived from or related to GAL4, CTF/NF1, AP2, ITF1, Oct1 and SpI.

A transrepressor domain is, for example, the *krab* transrepression domain of human Kruppel-associated box-protein. Other transrepressor domains considered are domains derived from or related to, for example, the v-erbA oncogenes product, the thyroid hormone receptor, the Ssn6/Tup1 protein complex, the SIRI protein, NeP1, TSF3, SF1, WT1, Oct-2.1, E4BP4, ZF5, any transcriptional regulator containing a PhD bromo domain and also those listed in US Patent 6,287,813.

A promoter considered is, for example, the constitutive simian virus 40 promoter (P_{SV40}), the minimal human cytomegalovirus immediate early promoter (P_{hCMVmin}), the constitutive human cytomegalovirus promoter (P_{hCMV}), the human elongation factor 1α promoter (P_{hEF1α}), the phosphoglycerate kinase promoter (P_{PGK}), the human ubiquitin promoter (P_{hUBC}) and the beta-actin promoter.

An endogenous or exogenous protein considered in the context of the vectors and mammalian cells of the invention is, e.g., a protein interacting with uric acid, such as metabolising uric acid, for example uricase (urate oxidase) converting uric acid and its salts into allantoin, or transporting uric acid and its salts, for example mammalian urate transporter protein, in particular human urate transporter protein 1 (URAT1), or a protein easily detectable allowing quantification of uric acid.

A protein easily detectable is, for example, human placental secreted alkaline phosphatase (SEAP), a fluorescent or enhanced fluorescent protein (e.g. GFP, RFP, YFP, and the like), secreted alpha amylase (SAMY), luciferase, beta-galactosidase, beta-lactamase, and glucoronidase.

In particular the invention relates to such a mammalian cell and to such vectors wherein the bacterial uric acid sensor-regulator is uricase sensor-regulator HucR from *Deinococcus radiodurans* R1, and the operator sequence is the corresponding operator sequence hucO from *Deinococcus radiodurans* R1.

If, for example, the protein interacting with uric acid is urate oxidase ("uricase"), such as the therapeutically applicable rasburicase from *Aspergillus flavus* marketed under the trade name Fasturec®, the corresponding particular mammalian cell as described hereinafter represents an auto-level gene control system responding to pathological uric acid levels switching off transgene expression at non-pathological and oxidative stress protecting uric acid levels in urate oxidase deficient mice (representing an approved disease model for human hyperuricemic diseases like gouty arthritis and tumor lysis syndrome).

Systemic uric acid is a strong radical scavenger and protects the body from oxidative stress by detoxification of radicals. Only at highly elevated levels urate crystallizes in the joints, the kidney and subcutaneous tissues and causes painful inflammations and diseases like hyperuricemia, kidney stones and gouty arthritis. In contrast, the complete removal of uric acid from the body by constitutive expression of urate oxidase may result in an increased radical-based cellular and tissue damage, especially of neuronal cells, which is also shown by the protective nature of uric acid for neurodegenerative diseases like Alzheimer and Parkinson's disease. Therefore the maintenance of sub-pathological metabolite levels is an important step forward to novel gene-therapeutic applications.

The particular synthetic mammalian uric acid homeostasis control network as described hereinafter results in a dose-dependent urate oxidase-based therapeutic response. In urate oxidase-deficient mice developing hyperuricemia with human-like symptoms, the designer circuit decreases blood uric acid concentration to stable sub-pathologic levels and reduces urate crystal deposits in the kidney of treated animals. This therapeutic network provides self-sufficient automatic control of uric acid homeostasis by preventing critical urate accumulation while preserving basal uric acid levels for oxidative stress protection.

The mammalian UREX system as described hereinafter may also be used in the production of protein therapeutics in bioreactor applications. The production of transgenic enzymes, like e.g. members of the enzyme class of oxidases / reductases, may result in the enzyme-based catalytic conversion of compounds in media used for maintenance and growth of cells, and cause the release of radicals. The induced oxidative stress may perturb the continuous product formation by inhibiting cell growth or reducing the product stability. When controlling the expression of such enzymes with the UREX system the product formation is induced by the addition of exogenous uric acid which functions as an inducer of the enzyme expression as well as protecting the cells from process-borne oxidative stress by binding and detoxifying the generated radicals.

The invention further relates to a mammalian cell comprising the mentioned vectors, either stably or transiently transfected with the described vectors, and to such mammalian cells in a nanocontainer or microcontainer, e.g. in encapsulated form. A nanocontainer may be a virus, preferably an attenuated virus, in particular a viral capsid, synthetic or semisynthetic nano- or microparticles, such as spheres or tubes of a suitable geometry to incorporate mammalian cells, and the nano- or microcontainers formed *in situ* by encapsulation of mammalian cells, for example with alginate-poly-L-lysine. A particular example of a suitable nano- or microcontainer is the hollow fibre manufactured under the trade name CELLMAX®.

The invention further relates to a mammal excluding man comprising a mammalian cell as described, in particular a mammalian cell in a nano- or microcontainer.

The invention further relates to a method of treating a disease caused by excess of uric acid or lack of uric acid using such a mammalian cell. Diseases considered are those caused by excess of uric acid, e.g. hyperuricemia, gout, Lesch-Nyhan syndrome, nephropathy, and tumor lysis syndrome, and diseases caused by an insufficient level of uric acid, such as Alzheimer, Parkinson, and corresponding cancers. In particular, the invention relates to a method of treatment of a patient suffering from a disease caused by excess or lack of uric acid wherein a mammalian cell in a nano- or microcontainer as described herein is implanted into a patient in need thereof.

### Preferred embodiments

In a particular exemplary embodiment of the invention, a particular synthetic circuitry enabling mammalian cells to sense and respond to uric acid is constructed, capitalizing on the uric acid-responsive interaction between HucR and hucO coordinating oxidative stress response in *Deinococcus radiodurans* R1 (Wilkinson, S.P. & Grove, A., *loc.cit*.).

This preferred embodiment of the invention is as follows: A uric acid-responsive expression network (UREX) is constructed by a multistep engineering strategy involving (i) modification of the HucR start codon (GTG→ATG) and fusion to a Kozak consensus sequence for maximum expression in mammalian cells (mHucR), (ii) N-terminal fusion of mHucR to the Krueppel-associated box protein domain (Bellefroid, E.J., Poncelet, D.A., Lecocq, P.J., Revelant, O. & Martial, J.A., Proc. Natl. Acad. Sci. USA 88, 3608-3612 (1991)), producing a chimeric mammalian urate-dependent transsilencer (mUTS, KRAB-mHucR) and (iii) a synthetic promoter assembled by placing eight (P_{UREX8}, P_{SV40}-hucO₈) tandem hucO modules downstream of a simian virus 40 promoter (P_{SV40}) (*Fig. 1a*). In the absence of uric acid mUTS binds hucO₈ and silences transcription from P_{UREX8} (*Fig. 1b*). However, in the presence of uric acid mUTS is released from hucO₈ thereby inducing SEAP (human placental secreted alkaline phosphatase) expression. Co-transfection of the constitutive human elongation factor 1α promoter (P_{hEF1α}□□driven mUTS expression vector pCK25 (P_{hEF1α}-mUTS-pA) with pCK9 (P_{UREX8}-SEAP-pA) into human cervical adenocarcinoma cells (HeLa), human embryonic kidney cells (HEK-293) and human fibrosarcoma cells (HT-1080) grown in uric acid-free medium showed that mUTS is able to bind and silence P_{UREX8}-driven SEAP expression up to 98% (*Fig. 1c*). Addition of 5 mM uric acid to transfected cultures triggeres the release of mUTS from hucO₈ and derepresses SEAP expression (*Fig. 1d*).

In particular the invention relates to a mammalian cell and to vectors wherein the bacterial uric acid sensor-regulator is uricase sensor-regulator HucR from *Deinococcus radiodurans* R1, and the operator sequence is the corresponding operator sequence hucO from *Deinococcus radiodurans* R1. Further preferred embodiments are those wherein the uricase sensor-regulator HucR is fused to a Kozak consensus sequence, and wherein the Krueppel-associated box protein domain is N-terminally fused to the uricase sensor-regulator HucR. Other preferred embodiments are those wherein the promoter is the simian virus 40 promoter, and wherein the vector comprising the preferred operator sequence hucO from *Deinococcus radiodurans* R1 comprises hucO in several copies, e.g. between 5 and 50 copies, preferably between 5 and 20 copies. Most preferred are the vectors pCK25 and pCK9, and vectors wherein the the codons for SEAP in pCK9 are replaced by codons for another detectable protein, e.g. for those easily detectable proteins listed above.

Because of the evolutionary loss of hepatic uricase by mutational silencing, humans depend on the urate-anion transporter URAT1 for effective renal urate clearance and maintenance of uric acid homeostasis (Enomoto, A. et al., Nature 417, 447-452 (2002)). Owing to the transporter's urate-uptake capacity ectopic expression of URAT1 may increase intracellular uric acid levels and consequently amplify UREX sensitivity. HeLa, HEK-293 and HT-1080 engineered for UREX-controlled SEAP expression and transfected with a constitutive URAT1 expression vector (pURAT1) reach significantly higher SEAP levels compared to URAT1-free cells and are particularly more sensitive within the urate range typically found in the human blood (200-400 µM) (*Fig. 1e**-g*). Indeed, 72 h after intraperitoneal implantation of microencapsulated HeLa engineered for URAT1 and UREX-controlled SEAP expression into urate oxidase-deficient (*uox*^{*-*/*-*}) mice, an established mouse model for human gouty arthritis (Wu, X. et al., Proc. Natl. Acad. Sci. USA 91, 742-746 (1994)), the synthetic urate sensor circuit is sufficiently sensitive to discriminate between mice developing hyperuricemia and urate nephropathy (high urate levels; 0.35 ± 0.15 U/L SEAP) and animals which received the licensed urate-reducing arthritis therapeutic allopurinol in their drinking water (low urate levels; 0.02 ± 0.10 U/L SEAP) (*Fig. 2a*). Control mice treated with cells constitutively producing SEAP show unaltered SEAP expression in the presence (low urate levels; 0.85 ± 0.10 U/L SEAP) or absence (high urate levels; 0.90 ± 0.15 U/L SEAP) of allopurinol.

In order to characterize adjustability and long-term reversibility of the mammalian uric acid sensor system the stable human cell line HEK-293_{UREX15} is constructed which is triple-transgenic for UREX-controlled SEAP and URAT1 expression. SEAP expression of HEK-293_{UREX15} can be precisely adjusted and shows a progressive increase in response to escalating concentrations of uric acid (*Fig. 2b*). Reversibility of UREX-controlled SEAP production is assessed by cultivating HEK-293_{UREX15} for 10 days while alternating uric acid concentrations from 0 mM to 5 mM every 72 h. UREX control is completely reversible and can be reset at any time without showing any expression memory effect on SEAP production (*Fig. 2c*).

As a precise and robust uric acid sensor UREX is suitable for therapeutic control of pathologic levels of this metabolite. For feedback-controlled reduction of uric acid levels in an autonomous and self-sufficient manner the uric acid sensor circuit needs to be linked to expression of a uricase/urate oxidase (Uox) which converts urate to the more soluble and renally secretable allantoin (Legoux, R. et al., J. Biol. Chem. 267, 8565-8570 (1992)). The cofactor-independent *Aspergillus flavus* uricase is codon-optimized for expression in mammalian cells (mUox). mUox reduces uric acid (0.5 mM) in the culture medium when constitutively expressed (pCK67, P_{UREX8}-mUox-pA) or controlled by mUTS (pCK67 with co-expressed pCK25, P_{UREX8}-mUox-pA, P_{hEF1α}-mUTS-pA). Urate reduction is more efficient when mUox is engineered for mammalian cell-based secretion by in-frame fusion to an immunoglobulin-derived secretion signal (Fluri, D. et al., J. Control. Release 131, 211-219 (2008), smUox; SSlgk-mUox; pCK65, P_{UREX8}-SS_{lgk}-mUox-pA) (*Fig. 3a*).

The dynamics of UREX-controlled uric acid metabolism is profiled during a 120 h cultivation of HeLa_{URAT1} engineered for constitutive mUTS and P_{UREX8}-driven smUox expression (*Fig. 3b*). Whereas isogenic HeLa_{URAT1} control cells transgenic for constitutive smUox expression (without mUTS co-expression) clear uric acid from the medium the urate levels of cell cultures harboring the synthetic UREX-smUox control circuit level out at 0.1 mM (a concentration known to provide oxidative-stress protection in a physiologic context) indicating that urate levels have fallen below a threshold concentration which is no longer able to induce UREX control and trigger further urate oxidation. These data confirm self-sufficient feedback control of uric acid levels by UREX-smUox and indicate that this synthetic circuit reduces pathologic urate levels into an oxidative stress-protective range in an auto-controlled manner.

When *uox*^{*-*/*-*} mice, developing hyperuricemia with human-like urate nephropathy and gouty arthritis symptoms in the absence of allopurinol, are treated with UREX-controlled smUox expressing cell implants, their urate levels in the serum and the urine drops to concentrations reached by standard allopurinol therapy while control animals (HeLa_{URAT1} cell implants, no allopurinol) accumulate urate and develop acute gouty arthritic symptoms (*Fig. 3c**,d*). Histologic analysis of haematoxylin/eosin stained kidney sections from all treatment groups confirm that UREX-smUox-based therapeutic cell implants reduce crystalline renal uric acid deposits in the proximal tubules and almost completely reverse the nephropathic tissue state (*Fig. 3e-g*).

Gouty arthritis is the most painful rheumatic disease. Due to a urate oxidase deficiency urate blood levels of humans are up to 50 times higher than in other mammals which increases the risk to develop hyperuricemic diseases. The fact that uric acid is an essential free radical scavenger and oxidative stress protectant important to prevent development of cancer, Parkinson's disease and multiple sclerosis makes gouty arthritis treatment particularly challenging as urate levels need to be accurately adjusted to prevent formation of uric acids crystal deposits while providing sufficient oxidative stress protection. There are two main therapeutic strategies for the treatment of urate-borne diseases in clinical use: (i) administration of xanthine oxidase inhibitors like allopurinol which blocks purine metabolism and prevents endogenous uric acid production or (ii) intravenous injection of recombinant *Aspergillus flavus* uricase (e.g., Rasburicase®) which promotes clearance of urate from the bloodstream. While Rasburicase® is mainly used to prevent the tumor lysis syndrome in high-risk cancer patients, allopurinol, which is on the market for over forty years, has severe side effects, cannot be taken during an acute attack of gout and has to be discontinued when hyperuricemia-induced nephropathy reaches an advanced state. Febuxostat (Uloric®), another xanthine oxidase inhibitor, has recently been licensed by the US Food and Drug Administration (FDA) for the treatment chronic hyperuricemia.

By functional connection of the human urate transporter with an engineered *D. radiodurans*-derived mammalian uric acid sensor and a sensor-controlled as well as secretion-engineered *A. flavus* urate oxidase a synthetic circuit is designed which provides self-sufficient automatic control of uric acid levels in mice. The network's unique auto-level urate control dynamics enables (i) seamless monitoring of uric acid levels in the blood, (ii) automatic induction of the core sensor unit at pathologic uric acids levels, (iii) prompt reduction of urate by sensor-controlled expression of clinically licensed urate oxidase and (iv) spontaneous shut-down when reaching basal physiologic urate levels needed for oxidative stress protection. UREX-based control of uric acid levels in a mouse model of human gouty arthritis shows that the synthetic circuit operates as expected and most importantly within the clinically relevant concentration range. In humans, painful inflammations resulting from monosodium urate crystals form at blood urate levels of above 6.8 mg/dl (Terkeltaub, R., Bushinsky, D.A. & Becker, M.A., *Arthritis Res. Ther.* **8,** S4 (2006)). The UREX sensor is able to capture such pathologic levels and trigger dose-dependent expression of secreted uricase which consequently reduces urate in the bloodstream of treated animals about 80% to 5 mg/dl. At a circulation level below 6 mg/dl urate crystal deposits are known to dissolve and patients are free of any clinical manifestation of gouty arthritis. Therefore, cell implants harboring a UREX-based network for self-sufficient auto-level control of uric acid levels in the bloodstream represent a prophylactic strategy or therapeutic intervention replacing periodic small-molecule administration in hyperuricemic diseases.

### Experimental part

### Vector Construction

Design details of all plasmids and oligonucleotides are provided in Table 1.

**Table 1: Plasmids used and designed**

| Plasmid | Description and Cloning Strategy^{a,b} | Reference or Source |
|---|---|---|
| pSEAP2-control | Vector for constitutive expression of SEAP (P_{SV40}-SEAP-pA) | Clontech, Carlsbad CA, USA |
| pZeoSV2 | Vector for constitutive expression of the zeocin-resistance determinant | Invitrogen, Basel, Switzerland |
| pWW29 | Vector for constitutive expression of MphR(A) (P_{hEF1α}-MphR(A)-pA) | Weber et al., Nat. Biotechnol. 20, 901-907 (2002) |
| pWW43 | Vector for constitutive expression of ET4 (P_{SV40}-ET4-pA; ET4, MphR(A)-KRAB) | Weber *et al., loc.cit.* |
| pWW56 | Vector for macrolide-inducible SEAP expression (P_{SV40}-ETR8-SEAP-pA) | Weber et al., loc.cit. |
| pURAT1 | Vector for constitutive expression of human | ImaGenes GmbH, |
| | URAT1 (P_{hCMV}-URAT1-pA) | Berlin, Germany; IMAGE ID: 5183650 |
| pBluescript -mHucR | Vector encoding a synthesized codon-optimized mammalian variant of the *Deinococcus radiodurans* HucR (mHucR) | |
| pBluescript -smUox | Vector encoding a synthesized secreted codon-optimized mammalian variant of *Aspergillus flavus* Uox (smUox). smUox contains an SS_{lgK} secretion signal fused 5' of the *A*. *flavus*-derived codon-optimized mammalian Uox (mUox, SS_{lgK}-mUox) | |
| pBluescript -hucO₈ | Vector containing a synthesized mHucR-specific octameric hucO (hucO₈) operator of *Deinococcus radiodurans* | |
| pCK9 | Vector for urate-inducible P_{UREX8}-driven SEAP expression (P_{UREX8}-SEAP-pA; P_{UREX8}, P_{SV40}-hucO₈). hucO₈ was excised from pBluescript-hucO₈ (*Hin*dIII/*Eco*RI) and cloned in sense orientation into pSEAP2-control (*Hin*dIII/*Eco*RI) | |
| pCK24 | Vector for constitutive expression of mHucR (P_{hEF1α}-mHucR-pA). mHucR was PCR-amplified from pBluescript-mHucR using oligonucleotides OCK20 (5'-ttgqcqcqcTCAGCCCGCATGGACAACGA-3') and OCK21 (5'-gctctagattaTACCCCCTGC TCCAGCCC-3'), restricted with *Eco*RI/*Bss*HII and cloned into pWW29 (*Bss*HII/*Xba*I) | |
| pCK25 | Vector for constitutive expression of the transrepressor KRAB-mHucR (P_{hEF1α}-KRAB-mHucR-pA). KRAB was PCR-amplified from pWW43 using oligonucleotides OCK22 (5'-ggaattccaccATGGATGCTAAGTCA CTAAC-3') and OCK23 (5'-ttggcgcgc CCAGAGATCATTCCTTGCC-3'), restricted with *Eco*RI/*Bss*HII and cloned into pCK24 (*Eco*RI/*Bss*HII) | |
| pCK65 | Vector encoding P_{UREX8}-driven smUox expression (P_{UREX8}-smUox-pA). smUox was excised from pBluescript-smUox (*Eco*RI/*Xba*I) and cloned into pCK9 (*Eco*RI/*Xba*I) | |
| pCK67 | Vector for urate-inducible P_{UREX8}-driven mUox expression (P_{UREX8}-mUox-pA). mUox was PCR-amplified from pBluescript-smUox using oligonucleotides OCK66 (5'-ggaattccaccatgTCCGCAGTAAAAG CAGCCCGCTAC-3') and OCK67 (5'-CAAGCTTGCATGCAGGCCTCT GC-3'), restricted with *Eco*RI/*Xba*I and cloned into pCK9 (*Eco*RI/*Xba*I) | |

Restriction endonuclease-specific sites are underlined in oligonucleotide sequences. Annealing basepairs contained in oligonucelotide sequences are shown in capital letters.

*Abbreviations:* **ET4,** macrolide-dependent transsilencer; **ETR8,** octameric operator module specific for MphR(A); **hucO**, HucR-specific operator; **hucO₈**, octameric operator module specific for HucR; **KRAB,** Krueppel-associated box protein; **mHucR,** *Deinococcus radiodurans* urate-dependent repressor codon optimized for expression in mammalian cells; **MphR(A),** *Escherichia coli* macrolide-dependent repressor; **mUox,** intracellular variant of the urate oxidase from *Aspergillus flavus* codon-optimized for expression in mammalian cells; **pA,** SV40-derived late polyadenylation site; **P**_{hCMV}, human cytomegalovirus immediate early promoter; **P**_{hEF1α}, human elongation factor 1α promoter; **P**_{SV40}, simian virus 40 promoter; **P**_{UREX8}, urate-inducible promoter containing hucO₈ (P_{SV40}-hucO₈); **SEAP,** human placental secreted alkaline phosphatase; **SS**_{lgκ}, secretion signal derived from the murine lgκ-chain V-12-C region; **smUox,** mUox engineered for secretion by mammalian cells; **URAT1,** human organic anion/urate transporter SLC22A12 (solute carrier family 22, member 12).

### Cell culture and transfections

Human cervical adenocarcinoma cells (HeLa, ATCC CCL-2), human embryonic kidney cells (HEK-293, ATCC CRL-1573) and human fibrosarcoma cells (HT-1080, ATCC CCL-121) are cultivated in Dulbecco's modified Eagle's medium (DMEM, Invitrogen, Basel, Switzerland, Cat. No. 52100-39) supplemented with 10% (v/v) fetal calf serum (FCS, PAN Biotech GmbH, Aidenbach, Germany, Cat. No. 3302, Lot No. P251110) and 1% (v/v) penicillin/streptomycin solution (P/S, PAN Biotech GmbH, Cat. No. P06-07100). All cell types are cultivated at 37°C in a humidified atmosphere containing 5% CO₂. HEK-293 are transfected using a standard CaPO₄-based protocol (Weber, W. et al., Nucleic Acids Res. 31, e71 (2003)). HeLa were also transfected according to this standard CaPO₄-based protocol, with the exception that the DNA precipitates are incubated with the cells for 12 hours before changing the medium. HT-1080 are transfected with FuGENE 6 (Roche Diagnostics AG, Basel, Switzerland, Cat. No. 11814443001) according to the supplier's procedure. Transfected cells are cultivated in DMEM supplemented with 10% (v/v) knockout serum replacement (KOSR, Invitrogen, Basel, Switzerland, Cat. No. 10828-028), 1% P/S and, optionally, with uric acid (Acros Organics, Geel, Belgium, Cat. No. 171290250) (standard medium). To establish uric acid-dependent dose-response characteristics, 3×10⁵ cells are seeded per well of a six-well plate (Thermo Fisher Scientific, Roskilde, Denmark) and transfected as described above. The cells are trypsinized (200 µl Trypsin [PAN Biotech GmbH, Cat. No. P10-023500], 5 min., 37°C), collected by centrifugation (2 min., 120×g) and resuspended in 1.5 ml standard medium. 100 µl of this cell suspension are transferred to individual wells of a 96-well plate, supplemented with different concentrations of uric acid and cultivated for 48 h before reporter gene expression is profiled in the culture supernatant.

### Construction of stable transgenic cell lines

HeLa-derived HeLa_{URAT1} cells, transgenic for the constitutive expression of the human renal urate-anion exchanger (URAT1), are constructed by co-transfection of pURAT1 (P_{hCMV}-URAT1-pA; ImaGenes, Berlin, Germany, IMAGE ID: 5183650) and pZeoSV2 (conferring resistance to zeocin; [InvivoGEN, San Diego, USA, Cat. No. ant-zn-5]) at a ratio of 10:1 (3 µg total DNA). After a 14-day selection period using 200 µg/ml (w/v) zeocin cells are clonaly expanded and individual clones are profiled for URAT1 expression using qRT-PCR. The cell line HeLa_{URAT1} is chosen for further experiments. The triple-transgenic HEK-293-derived HEK-293_{UREX15} cell line, enabling urate-inducible SEAP expression, is constructed by sequential co-transfection and clonal selection of (i) pCK9 (P_{UREX8}-SEAP-pA) and pCK25 (P_{hEF1α}-KRAB-mHucR-pA, also carrying a constitutive expression cassette conferring resistance to blasticidin) (ratio of 1:1, 14-day selection in DMEM containing 10% FCS and 20 µg/ml (w/v) blasticidin [InvivoGen, San Diego, USA, Cat. No. ant-bl-1]) and (ii) pURAT1 (P_{hCMV}-URAT1-pA) and pZeoSV2 (conferring resistance to Zeocin) (ratio of 10:1 [(3 µg total DNA], 14-day selection in DMEM containing 10% FCS, 20 µg/ml (w/v) blasticidin and 200 µg/ml (w/v) zeocin (InvivoGen, San Diego, CA, USA, Cat. No. ant-zn-5). Individual HEK-293_{UREX} clones are randomly picked and assessed for urate-triggered SEAP expression. HEK-293_{UREX15} is chosen for further studies.

### Analytical assays

SEAP levels are quantified in cell culture supernatants and mouse serum using a standard p-nitrophenylphosphate-based light absorbance time course (Berger, J., Hauber, J., Hauber, R., Geiger, R. & Cullen, B.R., Gene 66, 1-10 (1988)). Uric acid levels are assessed in cell culture supernatants, murine serum or urine using the Amplex® Red uric acid/uricase assay kit (Invitrogen, Basel, Switzerland, Cat. No. A22181) according to the manufacturer's protocol. Uric acid-containing samples are diluted in reaction buffer. The addition of uricase triggers the enzymatic conversion of uric acid to allantoin, CO₂ and H₂O₂ that reacts, in the presence of horseradish peroxidase stoichiometrically with Amplex Red reagent to generate the red-fluorescent oxidation product resorufin which can be quantified at 585 nm.

### In vivo methods

Urate oxidase-deficient mice (*uox*^{*-*/*-*}) developing hyperuricemia with human-like gouty arthritis symptoms (Wu, X. et al., loc.cit.) are used to validate the synthetic UREX-based uric acid control network *in vivo* (Charles River Laboratories, France, mouse strain: JAKS-2223). Since *uox*^{*-*/*-*} mice die within 4 weeks of age breeding and long-term maintenance requires addition of 150 µg/mL (w/v) allopurinol (Sigma, Cat. No. A8003) to the drinking water. Two weeks before implantation of UREX-transgenic cells the allopurinol treatment of urate oxidase-deficient mice is either continued or stopped to produce animal groups with low or high pathogenic uric acid levels in the bloodstream, respectively. HeLa_{URAT1}, transgenic for constitutive URAT1 expression are transiently co-transfected with pCK25 (P_{hEF1α}-mUTS-pA) and either pCK9 (P_{UREX8}-SEAP-pA), pSEAP2-control (P_{SV40}-SEAP-pA) or pCK65 (P_{UREX8}-smUox-pA) and then microencapsulated in 200 µm alginate-(poly-L-lysine)-alginate capsules (200 cells/capsule) using an Inotech Encapsulator Research IE-50R (EncapBioSystems Inc., Greifensee, Switzerland) according to the manufacturer's protocol and applying the following settings:
200 µm single nozzle, stirrer speed control set to 5 units, 20 ml syringe with a flow rate of 410 units, nozzle vibration frequency 1024 Hz, voltage for capsule dispersion 900 V. 700 µl PBS containing 2×10⁶ encapsulated cells (10⁴ capsules/mouse) are intraperitoneally injected into urate oxidase-deficient mice. Control mice are implanted with microencapsulated parental HeLa_{URAT1}. 48 h post-implantation the mice are transferred to a clean cage and the urine of each treatment group is sampled for 24 h. 72 h after implantation the mice are sacrificed, their blood is collected and the serum isolated in microtainer SST tubes (Beckton Dickinson, Plymouth, UK) according to the manufacturer's protocol. All experiments involving animals are performed according to the directive of the European Community Council (86/609/EEC).

### Histology

For microscopic analysis of uric acid deposits in the kidney of *uox*^{*-*/*-*} mice implanted with microencapsulated cells transgenic for constitutive URAT1 and UREX-controlled smUox expression treated animals are split into treatment groups receiving 150 µg/mL (w/v) or no allopurinol in their drinking water as described above. Seven days after implantation, the mice are sacrificed, their kidneys explanted and fixed in 4% (w/v) paraformaldehyde (Sigma, Cat. No. P6148) in PBS for 4 h. The kidneys are washed in 10% (w/v) sucrose (Sigma, Cat. No. S1888) in PBS for 90 min, trimmed, dehydrated and embedded in paraffin. 3 µm sections of the tissues produced using an Ultracut device (Zeiss, Feldbach, Switzerland) are transferred to gelatinized microslides and air-dried overnight at 37°C. After paraffin removal by submersion in xylene (3x10min), the tissues are rehydrated by sequential incubation (10 min) in decreasing ethanol concentrations (90%, 80%, 70%), rinsed twice in TBS (Tris-buffered saline; 50 mM Tris/HCI [pH 7.4], 100 mM sodium chloride) and stained with haematoxylin/eosin solution. The samples are visualized under polarized light to assess anisotropism using a Leica DMRBE microscope.

## Claims

1. A mammalian cell useful in detecting and/or degrading a harmful excess of uric acid comprising
(a) a vector comprising a genetic code for a bacterial uric acid sensor-regulator fused to a transrepressor domain, wherein the bacterial uric acid sensor-regulator is uricase sensor-regulator HucR from *Deinococcus radiodurans* R1, the MarR type transcriptional regulator Dgeo_2531 from *Deinococcus geothermalis* DSM 11300, the MarR-type transcriptional regulator from *Pseudomonas mendocina* ypm, the MarR-type transcriptional regulator Deide_3p00280 from *Deinococcus deserti* VCD115, or a sensor-regulator derived therefrom, wherein the sensor-regulator derived therefrom contains only conservative amino substitutions and remains at least 90% identical to the said bacterial uric acid sensor-regulator at the amino acid level and includes compounds at the polynucleotide level comprising triplet codons coding for the same amino acid but being especially adapted to mammalian cells; and
(b) a vector comprising an operator sequence specifically binding a bacterial uric acid sensor-regulator, a promoter and a polynucleotide coding for a protein, wherein the operator sequence is an operator sequence produced by *Deinococcaceae or Pseudomonadaceae* specifically binding to sensor-regulator HucR from *Deinococcus radiodurans* R1, Dgeo_2531 from *Deinococcus geothermalis* DSM 11300, the MarR-type transcriptional regulator from *Pseudomonas mendocina* ypm, or Deide_3p00280 from *Deinococcus deserti* VCD115, or an operator sequence derived therefrom, wherein the operator sequence derived therefrom is an operator sequence produced by *Deinococcaceae or Pseudomonadaceae* specifically binding to sensor-regulator HucR from *Deinococcus radiodurans* R1, Dgeo_2531 from *Deinococcus geothermalis* DSM 11300, the MarR-type transcriptional regulator from *Pseudomonas mendocina* ypm, or Deide_3p00280 from *Deinococcus deserti* VCD115, wherein one to twelve nucleotides of the operator sequence are replaced by other nucleotides without diminishing the intended interaction with the sensor-regulator, wherein the protein is uricase or urate transporter protein.

2. The mammalian cell according to claim 1 wherein the bacterial uric acid sensor-regulator is uricase sensor-regulator HucR from *Deinococcus radiodurans* R1.

3. The mammalian cell according to claim 1 or 2 wherein the transrepressor domain is selected from the group consisting of the *krab* transrepression domain of human Kruppel-associated box-protein and the transrepressor domains derived from or related to the v-erbA oncogenes product, the thyroid hormone receptor, the Ssn6/Tup1 protein complex, the SIRI protein, NeP1, TSF3, SF1, WT1, Oct-2.1, E4BP4, and ZF5.

4. The mammalian cell according to any one of claims 1 to 3 wherein the operator sequence is the operator sequence hucO from *Deinococcus radiodurans* R1.

5. The mammalian cell according to any one of claims 1 to 4 wherein the promoter is selected from the group consisting of the constitutive simian virus 40 promoter (P_{SV40}), the minimal human cytomegalovirus immediate early promoter (P_{hCMVmin}), the constitutive human cytomegalovirus promoter (P_{hCMV}), the human elongation factor 1α promoter (P_{hEF1α}), the phosphoglycerate kinase promoter (P_{PGK}), the human ubiquitin promoter (P_{hUBC}) and the beta-actin promoter.

6. A mammalian cell according to any one of claims 1 to 5, which is a HeLa-derived, HEK-293-derived or HT-1080-derived cell.

7. A nano- or microcontainer comprising the mammalian cell according to any one of the claims 1 to 5.

8. A mammal excluding a human being comprising the mammalian cell according to any one of the claims 1 to 5.

## Patentansprüche

1. Säugerzelle, die zum Nachweisen und/oder Abbauen eines schädlichen Überschusses an Harnsäure geeignet ist und Folgendes umfasst
(a) einen Vektor, der einen genetischen Code für einen bakteriellen Harnsäure-Sensor-Regulator, fusioniert an eine Transrepressordomäne, umfasst, wobei es sich bei dem bakteriellen Harnsäure-Sensor-Regulator um Uricase-Sensor-Regulator HucR aus *Deinococcus radiodurans* R1, den MarR-Typ-Transkriptionsregulator Dgeo_2531 aus *Deinococcus geothermalis* DSM 11300, den MarR-Typ-Transkriptionsregulator aus *Pseudomonas mendocina* ypm, den MarR-Typ-Transkriptionsregulator Deide_3p00280 aus *Deinococcus deserti* VCD115 oder einen davon stammenden Sensor-Regulator handelt, wobei der davon stammende Sensor-Regulator nur konservative Aminosäuresubstitutionen enthält und zu mindestens 90% mit dem bakteriellen Harnsäure-Sensor-Regulator auf Aminosäureebene identisch bleibt und auf der Polynukleotidebene Verbindungen enthält, die Triplettcodons umfassen, die für dieselbe Aminosäure codieren, aber speziell an Säugetierzellen angepasst sind; und
(b) einen Vektor, der eine Operatorsequenz, die spezifisch einen bakteriellen Harnsäure-Sensor-Regulator bindet, einen Promotor und ein für ein Protein codierendes Polynukleotid umfasst, wobei es sich bei der Operatorsequenz um eine von *Deinococcaceae* oder *Pseudomonadaceae* produzierte Operatorsequenz handelt, die spezifisch an Sensor-Regulator HucR aus *Deinococcus radiodurans* R1, Dgeo_2531 aus *Deinococcus geothermalis* DSM 11300, den MarR-Typ-Transkriptionsregulator aus *Pseudomonas mendocina* ypm oder Deide_3p00280 aus *Deinococcus deserti* VCD 115 oder eine davon stammende Operatorsequenz bindet, wobei die davon stammende Operatorsequenz eine von *Deinococcaceae* oder *Pseudomonadaceae* produzierte Operatorsequenz ist, die spezifisch an Sensor-Regulator HucR aus *Deinococcus radiodurans* R1, Dgeo_2531 aus *Deinococcus geothermalis* DSM 11300, den MarR-Typ-Transkriptionsregulator aus *Pseudomonas mendocina* ypm oder Deide_3p00280 aus *Deinococcus deserti* VCD 115 bindet, wobei ein bis zwölf Nukleotide der Operatorsequenz durch andere Nukleotide ersetzt sind, ohne die beabsichtigte Wechselwirkung mit dem Sensor-Regulator zu verringern, wobei das Protein Uricase oder Urat-Transporterprotein ist.

2. Säugerzelle nach Anspruch 1, wobei der bakterielle Harnsäure-Sensor-Regulator Uricase-Sensor-Regulator HucR aus *Deinococcus radiodurans* R1 ist.

3. Säugerzelle nach Anspruch 1 oder 2, wobei die Transrepressordomäne aus der Gruppe ausgewählt ist, bestehend aus der *krab*-Transrepressionsdomäne von humanem Krüppel-assoziiertem Box-Protein und den Transrepressordomänen, die von dem v-erbA-Onkogenprodukt, dem Schilddrüsenhormonrezeptor, dem Ssn6/Tup1-Proteinkomplex, dem SIRl-Protein, NeP1, TSF3, SF1, WT1, Oct-2.1, E4BP4 und ZF5 stammen oder damit verwandt sind.

4. Säugerzelle nach einem der Ansprüche 1 bis 3, wobei es sich bei der Operatorsequenz um die Operatorsequenz hucO aus *Deinococcus radiodurans* R1 handelt.

5. Säugerzelle nach einem der Ansprüche 1 bis 4, wobei der Promotor aus der Gruppe ausgewählt ist, bestehend aus dem konstitutiven Simianvirus 40-Promotor (P_{SV40}), dem minimalen Immediate-Early-Promotor des humanen Cytomegalievirus (P_{hCMVmin}), dem konstitutiven Promotor des humanen Cytomegalievirus (P_{hCMV}), dem Promotor des humanen Elongationsfaktors 1α (P_{hEF1α}), dem Phosphoglyceratkinase-Promotor (P_{PGK}), dem humanem Ubiquitin-Promotor (P_{hUBC}) und dem beta-Aktin-Promotor.

6. Säugerzelle nach einem der Ansprüche 1 bis 5, die eine von HeLa stammende, von HEK-293 stammende oder von HT-1080 stammende Zelle ist.

7. Nano- oder Mikrobehälter, der die Säugerzelle nach einem der Ansprüche 1 bis 5 umfasst.

8. Säuger, mit Ausnahme von Mensch, der die Säugerzelle einem der Ansprüche 1 bis 5 umfasst.

## Revendications

1. Cellule mammifère utile dans la détection et/ou la dégradation d'un excès nocif d'acide urique comprenant
(a) un vecteur comprenant un code génétique pour un détecteur-régulateur d'acide urique bactérien fusionné à un domaine transrépresseur, dans laquelle le détecteur-régulateur d'acide urique bactérien est le détecteur-régulateur de l'uricase HucR de *Deinococcus radiodurans* R1, le régulateur transcriptionnel de type MarR Dgeo_2531 de *Deinococcus geothermalis* DSM 11300, le régulateur transcriptionnel de type MarR de *Pseudomonas mendocina* ypm, le régulateur transcriptionnel de type MarR Deide_3p00280 de *Deinococcus deserti* VCD115, ou un détecteur-régulateur dérivé de ceux-ci, dans laquelle le détecteur-régulateur dérivé de ceux-ci ne présente que des substitutions conservatrices d'acides aminés et reste identique à au moins 90 % audit détecteur-régulateur d'acide urique bactérien au niveau des acides aminés et comprend des composés au niveau polynucléotidique comprenant des codons (triplets) codant pour le même acide aminé, mais se montrant particulièrement bien adaptés aux cellules mammifères ; et
(b) un vecteur comprenant une séquence opérateur se liant de façon spécifique à un détecteur-régulateur d'acide urique bactérien, un promoteur et un polynucléotide codant pour une protéine, dans laquelle la séquence opérateur est une séquence opérateur produite par des *Deinococcaceae* ou des *Pseudomonadaceae* se liant de façon spécifique au détecteur-régulateur HucR de *Deinococcus radiodurans* R1, à Dgeo_2531 de *Deinococcus geothermalis* DSM 11300, au régulateur transcriptionnel de type MarR de *Pseudomonas mendocina* ypm, ou à Deide_3p00280 de *Deinococcus deserti* VCD115, ou une séquence opérateur dérivée de celles-ci, dans laquelle la séquence opérateur dérivée de celles-ci est une séquence opérateur produite par des *Deinococcaceae* ou des *Pseudomonadaceae* se liant de façon spécifique au détecteur-régulateur HucR de *Deinococcus radiodurans* R1, à Dgeo_2531 de *Deinococcus geothermalis* DSM 11300, au régulateur transcriptionnel de type MarR de *Pseudomonas mendocina* ypm, ou à Deide_3p00280 de *Deinococcus deserti* VCD115, dans laquelle un à douze nucléotides de la séquence opérateur sont remplacés par d'autres nucléotides sans réduction de l'interaction prévue avec le détecteur-régulateur, dans laquelle la protéine est l'uricase ou la protéine de transport des urates.

2. Cellule mammifère selon la revendication 1, dans laquelle le détecteur-régulateur d'acide urique bactérien est le détecteur-régulateur de l'uricase HucR de *Deinococcus radiodurans* R1.

3. Cellule mammifère selon la revendication 1 ou 2, dans laquelle le domaine transrépresseur est choisi dans le groupe constitué du domaine de transrépression *krab* de la protéine humaine de la boîte associée Krüppel et des domaines transrépresseurs dérivés du produit des oncogènes v-erbA, du récepteur de l'hormone thyroïdienne, du complexe protéique Ssn6/Tup1, de la protéine SIRI, de NeP1, TSF3, SF1, WT1, Oct-2.1, E4BP4 et ZF5, ou associés à ceux-ci.

4. Cellule mammifère selon l'une quelconque des revendications 1 à 3, dans laquelle la séquence opérateur est la séquence opérateur hucO de *Deinococcus radiodurans* R1.

5. Cellule mammifère selon l'une quelconque des revendications 1 à 4, dans laquelle le promoteur est choisi dans le groupe constitué du promoteur constitutif du virus simien 40 (P_{SV40}), du promoteur précoce immédiat de cytomégalovirus humain minimal (P_{hCMVmin}), du promoteur constitutif du cytomégalovirus humain (P_{hCMV}), du promoteur du facteur d'élongation 1α humain (P_{hEF1α}), du promoteur de la phosphoglycérate kinase (P_{PGK}), du promoteur de l'ubiquitine humaine (P_{hUBC}) et du promoteur de la bêta-actine.

6. Cellule mammifère selon l'une quelconque des revendications 1 à 5, qui est une cellule dérivée des cellules HeLa, HEK-293 ou HT-1080.

7. Nano- ou micro-récipient comprenant la cellule mammifère selon l'une quelconque des revendications 1 à 5.

8. Mammifère autre qu'un être humain comprenant la cellule mammifère selon l'une quelconque des revendications 1 à 5.
